(19) Europäisches Patentamt
European Patent Office
Office européen des brevets

(11) Veröffentlichungsnummer: **0 226 119 B1**

(12) # EUROPÄISCHE PATENTSCHRIFT

(45) Veröffentlichungstag der Patentschrift: **23.05.90**

(51) Int. Cl.$^5$: **C 07 C 53/21**

(21) Anmeldenummer: **86116765.8**

(22) Anmeldetag: **02.12.86**

(54) **Neue Halogen-tetrafluorpropionsäure, Verfahren zu ihrer Herstellung und ihre Verwendung.**

(30) Priorität: **11.12.85 DE 3543710**

(43) Veröffentlichungstag der Anmeldung:
**24.06.87 Patentblatt 87/26**

(45) Bekanntmachung des Hinweises auf die Patenterteilung:
**23.05.90 Patentblatt 90/21**

(84) Benannte Vertragsstaaten:
**DE FR GB IT**

(56) Entgegenhaltungen:
**DE-A-2 120 364**

(73) Patentinhaber: **HOECHST AKTIENGESELLSCHAFT**
**Postfach 80 03 20**
**D-6230 Frankfurt am Main 80 (DE)**

(72) Erfinder: **Von Werner, Konrad, Dr.**
**Buch 1 1/2**
**D-8261 Halsbach (DE)**
Erfinder: **Probst, Anton**
**Gerhart-Hauptmann-Strasse 5**
**D-8269 Burgkirchen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 226 119 B1

Courier Press, Leamington Spa, England.

**Beschreibung**

Die Erfindung betrifft ein Verfahren zur Herstellung von 3-Iod-tetrafluorpropionsäure.

3-Halogen-tetrafluorpropionsäuren, nämlich die 3-Brom- und die 3-Chlor-tetrafluorpropionsäure sowie die Pentafluorpropionsäure sind bereits bekannt (vgl. Chemical Abstracts, Vol. 70, 1969, Abstr. Nr. 77334q).

Ihre Herstellung erfolgt durch eine thermische Additionsreaktion, Abspaltung von Halogenwasserstoffsäure aus der Additionsverbindung und anschließende Oxidation zur entsprechenden Propionsäure; die nachstehenden Reaktionsgleichungen, in denen X für Brom steht, sollen dies veranschaulichen:

$$XCF_2CF_2X + CH_2 = CH_2 \xrightarrow[\text{Addition}]{\text{thermische}} XCF_2CF_2CH_2CH_2X$$

$$XCF_2CF_2CH_2CH_2X \xrightarrow{-HX} XCF_2CF_2{-}CH{=}CH_2$$

$$XCF_2CF_2{-}CH{=}CH_2 \xrightarrow{\text{Oxidation}} XCF_2CF_2{-}COOH.$$

Da das Bromatom und insbesondere das Chlor- und Fluoratom relativ stark gebunden und deshalb mehr oder weniger reaktionsträge sind, sind die bekannten 3-Halogen-tetrafluorpropionsäuren weitgehend ungeeignet als Ausgangsprodukte zur Herstellung von Verbindungen, die anstelle des Halogenatoms eine vorteilhafte funktionelle Gruppe aufweisen.

Die 3-Iod-tetrafluorpropionsäure (3-Iod-2.2-3.3-tetrafluorpropionsäure) würde diese Nachteile nicht aufweisen.

Aus dem US-Patent 3 016 407 ist ein Verfahren zur Herstellung der Verbindungen $ICF_2CF_2CH_2CH_2I$ und $ICH_2CH_2CF_2CF_2CH_2CH_2I$ durch thermische Addition von 1.2-Diiod-tetrafluorethan ($ICF_2CF_2I$) an Ethylen bekannt. Dabei wird $ICF_2CF_2I$ und $CH_2{=}CH_2$ unter Druck auf eine Temperatur von 180 bis 220°C erhitzt und aus dem erhaltenen Reaktionsprodukt wird die angestrebte Verbindung gewonnen. Im Falle einer diskontinuierlichen Durchführung des Verfahrens wird empfohlen, zunächst $ICF_2CF_2I$ herzustellen durch Reaktion von $I_2$ und $C_2F_4$ bei einer Temperatur von etwa 150°C, und anschließend in einer zweiten Reaktionsstufe die Umsetzung von $ICF_2CF_2I$ mit Ethylen vorzunehmen. In der genannten US—PS wird ausdrücklich hervorgehoben, daß die Herstellung von $ICF_2CF_2CH_2CH_2I$ durch gleichzeitige Umsetzung von Iod, Tetrafluorethen und Ethen, nicht möglich ist, weil man dabei beträchtliche Mengen an unerwünschtem 1.2-Diiod-ethan ($ICH_2CH_2I$) erhalten würde.

Es wurde nun überraschenderweise gefunden, daß sich die Verbindung $ICF_2CF_2CH_2CH_2I$ im Eintopfverfahren aus einem Gemisch von Iod, Tetrafluorethen und Ethen in guten Ausbeuten herstellen läßt, ohne daß sich dabei die Verbindung $ICH_2CH_2I$ in nennenswerten Mengen bildet. Es wurde ferner gefunden, daß aus der genannten Butanverbindung durch Abspaltung von Iodwasserstoffsäure die Verbindung $ICF_2CF_2{-}CH{=}CH_2$ und daraus durch Oxidation die entsprechende Propionsäure gewonnen werden kann.

Das erfindungsgemäße Verfahren zur Herstellung von 3-Iod-tetrafluorpropionsäure ist dadurch gekennzeichnet, daß man in einer Eintopfreaktion ein Gemisch von Iod, Tetrafluorethen und Ethen in einem Molverhältnis von 1:(1,2—2):(1—1,2) bei einer Temperatur von 170 bis 200°C umsetzt und aus dem erhaltenen Reaktionsprodukt die Verbindung 1.4-Diiod-1.1.2.2-tetrafluorbutan ($ICF_2CF_2CH_2CH_2I$) gewinnt, diese Butan-Verbindung zur Abspaltung von Iodwasserstoff (HI) mit einer Base umsetzt und aus diesem Reaktionsprodukt die Verbindung 1-Iod-1.1.2.2-tetrafluor-3-buten ($ICF_2CF_2{-}CH{=}CH_2$) gewinnt, diese Buten-Verbindung zur Überführung in die entsprechende Propionsäure mit einem Oxidationsmittel umsetzt und aus diesem Reaktionsprodukt die 3-Iod-2.2.3.3-tetrafluorpropionsäure ($ICF_2CF_2COOH$) gewinnt.

Dem erfindungsgemäßen Verfahren entsprechen die nachstehenden Reaktionsgleichungen (1) bis (3):

$$I_2 + CF_2{=}CF_2 + CH_2{=}CH_2 \rightarrow ICF_2CF_2CH_2CH_2I \qquad (1)$$

$$ICF_2CF_2CH_2CH_2I \xrightarrow{-HI} ICF_2CF_2{-}CH{=}CH_2 \qquad (2)$$

$$ICF_2CF_2{-}CH{=}CH_2 \xrightarrow{\text{Oxidation}} ICF_2CF_2{-}COOH \qquad (3)$$

Die Umsetzung gemäß Reaktionsgleichung (1) ist möglich und führt auch zu hohen Ausbeuten der angestrebten Verbingung, wenn man die oben angegebenen erfindungsgemäßen Bedingungen einhält. Ein größerer Überschuß an Tetrafluorethen als die angegebenen 1,2 bis 2 mol begünstigt die Bildung von $ICF_2CF_2I$ und $ICH_2CH_2(CF_2CF_2)_nI$, ein größerer Überschuß an Ethen als die angegebenen 1,2 mol begünstigt die Bildung von $ICH_2CH_2I$ und $ICH_2CH_2CF_2CF_2CH_2CH_2I$ (n = eine ganze Zahl >1). Die erforderliche

Reaktionszeit bei der angegebenen Reaktionstemperatur von 170 bis 200°C beträgt je nach gewählter Temperatur etwa 5 bis 20 Stunden, das heißt, die Reaktionszeit bis zum praktisch vollständigen Umsatz liegt im Falle von 200°C Reaktionstemperatur bei etwa 5 Stunden und im Falle von 170°C Reaktionstemperatur bei etwa 20 Stunden. Während der erfindungsgemäßen Umsetzung herrscht klarerweise ein den vorliegenden Verbindungen entsprechender Druck. Er beträgt zu Beginn der Umsetzung etwa 40 bis 70 bar und fällt mit fortschreitender Umsetzung ab. Aus dem erhaltenen Reaktionsprodukt wird die angestrebte Verbindung gewonnen. Dies erfolgt vorzugsweise durch fraktionierte Destillation des flüssigen Reaktionsproduktes. Bei Einhaltung der erfindungsgemäßen Reaktionsbedingungen wird nicht nur eine hohe Ausbeute an angestrebter Verbindung ($ICF_2CF_2CH_2CH_2I$) erhalten, es werden auch keine oder nur sehr wenig Nebenprodukte wie Diiodethan ($ICH_2CH_2I$) gebildet.

Bei der Umsetzung nach Reaktionsgleichung (2) wird mit Hilfe einer Base Iodwasserstoff abgespalten. Die Art der Base ist nicht kritisch. Beispiele für geeignete Basen sind Alkalimetallhydroxide, wie Ätzkali und Ätznatron, Alkoxide wie Natriummethylat und Kaliummethylat oder tertiäre Amine. Nach einer bevorzugten Durchführung der Reaktionsgleichung (2) wird die Verbindung $ICF_2CF_2CH_2CH_2I$ mit einer mindestens stöchiometrischen Menge an Kalium- oder Natriumhydroxid in Form einer etwa 10 bis 60 gew.-%igen wäßrigen Lösung bei einer Temperatur von 20 bis 100°C, vorzugsweise 50 bis 90°C, umgesetzt (die Umsetzung kann an der entstehenden Menge Kaliumiodid beziehungsweise Natriumiodid verfolgt werden) und aus dem Reaktionsprodukt die angestrebte Verbindung ($ICF_2CF_2—CH=CH_2$) durch Phasentrennung oder fraktionierte Destillation gewonnen. Es ist vorteilhaft, einen Überschuß (im Vergleich zur stöchiometrischen Menge) an Ätzkali oder Ätznatron einzusetzen, und zwar einen Überschuß von etwa 0,5 bis 2 mol.

Es wurde gefunden, daß die angegebene Umsetzung mit Alkalilauge dann besonders vorteilhaft abläuft und beschleunigt wird, wenn Phasentransfer-Katalysatoren z.B. in Form von Tetraalkylammoniumsalzen anwesend sind. Beispiele für geeignete Tetraalkylammoniumsalze sind Tetrabutylammoniumbromid, Tricetylmethylammoniumchlorid, Dioctyldimethylammoniumchlorid und Dodecyltrimethylammoniumhydrogensulfat. Die Menge an Phasentransfer-Katalysator beträgt etwa 0,1 bis 5 Mol-%, vorzugsweise 0,5 bis 2 Mol-%, bezogen auf die Menge an eingesetzter Verbindung $ICF_2CF_2CH_2CH_2I$. Bei der angegebenen Umsetzung mit Alkalilauge ist es zweckmäßig, den Phasentransfer-Katalysator der Alkalilauge zuzusetzen und die Verbindung $ICF_2CF_2CH_2CH_2I$ der vorgelegten Lauge mit dem Phasentransfer-Katalysator bei der angegebenen Temperatur zuzudosieren.

Die Oxidation nach Reaktionsgleichung (3) wird mit Hilfe eines Oxidationsmittels durchgeführt. Die Art des Oxidationsmittels ist nicht kritisch. Beispiele für geeignete Oxidationsmittel sind Kaliumpermanganat, Chromtrioxid, Chromschwefelsäure, Osmiumtetroxid, Ozon und Peroxoessigsäure. Nach einer bevorzugten Durchführung der Reaktionsgleichung (3) wird die Verbindung $ICF_2CF_2—CH=CH_2$ mit einer mindestens stöchiometrischen Menge an Kaliumpermanganat in Form einer etwa 10 bis 40 gew.-%igen wäßrigen Lösung oder Suspension bei einer Temperatur von 5 bis 50°C, vorzugsweise 10 bis 25°C, umgesetzt (die Umsetzung kann am Verbrauch des Oxidationsmittels verfolgt werden) und aus dem Reaktionsprodukt nach Abtrennen des entstandenen Braunsteins und Ansäuern die angestrebte 3-Iod-tetrafluorpropionsäure durch fraktionierte Destillation gewonnen. Es ist zweckmäßig, einen geringen Überschuß (im Vergleich zur stöchiometrischen Menge) an Kaliumpermanganat einzusetzen, und zwar einen Überschuß von etwa 0,01 bis 0,1 mol. Die nachstehende Reaktionsgleichung soll die beschriebene Oxidation mit Kaliumpermanganat veranschaulichen:

$$ICF_2CF_2—CH=CH + 10\ KMnO_4 \rightarrow 3\ ICF_2CF_2—CO_2K + 10\ MnO_2 + 3\ K_2CO_3 + KOH + 4\ H_2O$$

Es wurde gefunden, daß die beschriebene Oxidation mit einer wäßrigen Kaliumpermanganatlösung dann besonders hohe Ausbeuten an der angestrebten Propionsäure bringt, wenn Phasentransfer-Katalysatoren z.B. in Form von quartären Ammonium- oder Phosphoniumsalzen anwesend sind. Die oben angegebenen Tetraalkylammoniumsalze und die entsprechenden Phosphoniumsalze stellen auch hier geeignete Phasentransfer-Katalysatoren dar. Die Menge an Phasentransfer-Katalysator beträgt etwa 0,5 bis 5 Mol-%, vorzugsweise 1 bis 3 Mol-%, bezogen auf die Menge an eingesetzter Verbindung $ICF_2CF_2—CH=CH_2$. Bei der oben angegebenen Umsetzung wird zweckmäßigerweise so vorgegangen, daß man die wäßrige Kaliumpermanganatlösung mit dem zugesetzten Phasentransfer-Katalysator vorlegt und die Verbindung $ICF_2CF_2—CH=CH_2$ bei der angegebenen Temperatur zudosiert. Die Reaktionsgeschwindigkeit läßt sich bequem über die Dosiergeschwindigkeit steuern, mit der das Olefin der gerührten Mischung aus Kaliumpermanganat, Wasser und Phasentransfer-Katalysator zugeführt wird. Die Reaktionswärme kann leicht durch Kühlung beherrscht werden. Am Ende der Reaktion liegt die 3-Iod-tetrafluorpropionsäure in Form ihres Kaliumsalzes neben Braunstein vor. Das Kaliumsalz kann durch Heißfiltration vom Braunstein ($MnO_2$) abgetrennt werden und die freie Propionsäure durch Eindampfen des Filtrates zur Trockne, Zugabe von vorzugsweise konzentrierter Schwefelsäure und Destillation gewonnen werden. Die 3-Iod-tetrafluorpropionsäure ($ICF_2CF_2—COOH$) ist eine farblose Flüssigkeit, die sich am Licht langsam rosa färbt und bei Raumtemperatur kristallisiert. Sie hat bei 13 mbar einen Siedepunkt von 78°C.

Die 3-Iod-tetrafluorpropionsäure ist eine wertvolle Ausgangsverbindung für die Herstellung anderer Ω-Iod-Verbindungen, die am endständigen Iod-Atom nach bekannten Verfahren funktionalisiert werden

können. So ist die neue 3-Halogen-tetrafluorpropionsäure eine vorteilhafte Ausgangsverbindung für die Herstellung von funktionalisierten, ungesättigten Comonomeren für die Bereitung von Elektrolyse-Membranen. So kann man aus $ICF_2CF_2COOH$ mit Hilfe bekannter Methoden beispielsweise zur Verbindung $FSO_2—(CF_2)_3—O—CF=CF_2$ kommen. Diese Verbindung und ihre vorteilhafte Verwendung zur Herstellung von Polymeren zur Bereitung von Elektrolyse-Membranen ist bekannt.

Die Erfindung wird nun an einem Beispiel noch näher erläutert.

Die im Beispiel angegebenen Kernresonanzdaten beziehen sich auf Deuterochloroform-Lösungen (Standard: Tetramethylsilan für $^1H$—NMR, Trifluoressigsäure für $^{19}F$—NMR. Signale mit positivem Vorzeichen liegen bei tieferem Feld gegenüber der Standard-Resonanz).

Herstellung von 1.4-Diiod-1.1.2.2-tetrafluor-butan

Ein 250 ml-Schüttelautoklav wird mit 76,2 g (0,3 mol) Iod beschickt, auf −76°C gekühlt und durch dreimaliges Evakuieren und Füllen mit Argon von Sauerstoff befreit. Nach erneutem Evakuieren werden nacheinander 50 g (0,5 mol) Tetrafluorethen und 9,0 g (0,32 mol) Ethen einkondensiert. Der Autoklav wird nun in 5 Stunden auf 180°C aufgeheizt und 15 Stunden bei dieser Temperatur geschüttelt. Der Druck fällt dabei von 58 bar auf 14 bar. Nach Abkühlen auf Raumtemperatur und Entspannen erhält man 121,3 g eines flüssigen Produkts, welches nach Kernresonanz-Analyse ($^{19}F$- und $^1H$-NMR) folgende Zusammensetzung (in Mol-%) aufweist:

0,9% $ICH_2CH_2I$
6,5% $ICF_2CF_2I$
3,0% $ICF_2CF_2H$
80,1% $ICF_2CF_2CH_2CH_2I$,
4,3% $I(CF_2CF_2)_2CH_2CH_2I$,
5,2% $ICH_2CH_2CF_2CF_2CH_2CH_2I$.

Durch fraktionierte Destillation dieses Gemisches im Vakuum erhält man 88,5 g $ICF_2CF_2CH_2CH_2I$ (Flüssigkeit mit Kp. 72°C/15 mbar; färbt sich am Licht violett). Die Ausbeute beträgt 77,3%, bezogen auf das eingesetzte Iod. Kernresonanzdaten:

$^{19}F$-NMR: 18,8 ppm ($CF_2I$), −29,3 ppm ($CF_2$).
$^1H$-NMR: 3,24 ppm ($CH_2I$, Triplett), 2.74 ppm ($CF_2$, Multiplett).

Herstellung von 1-Iod-1.1.2.2-tetrafluor-3-buten

In einem 2,5 Liter-Glaskolben mit Tropftrichter, Rührer, Thermometer und aufgesetzter 20 cm-Füllkörperkolonne werden 200 g (5,0 mol) NaOH, 1 Liter entsalztes Wasser und 7,5 g (0,025 mol) $[(C_8H_{17})_2N(CH_3)_2]^+Cl^-$ als Phasentransfer-Katalysator vorgelegt. Die Mischung wird auf 90°C erwärmt und in die so erwärmte Mischung werden 819 g (2,14 mol) $ICF_2CF_2CH_2CH_2I$ unter Rühren langsam zugetropft, wobei man das Reaktionsgemisch am Sieden hält. Man stellt das Ablauf/Rücklauf-Verhältnis am Kopf der Kolonne auf 1:1 ein und fängt kontinuierlich in einer eisgekühlten Vorlage ein Gemisch aus Fluorverbindungen und Wasser auf, so lange, bis keine organischen Anteile mehr übergehen (Sumpftemperatur am Schluß 105°C; Reaktionsdauer: 2,5 h). Das Destillat wird mit Wasser ausgeschüttelt und die organische Phase mit Natriumsulfat getrocknet. Man erhält 451 g Rohprodukt, dessen fraktionierte Destillation 335 g (1,319 mol) $ICF_2CF_2—CH=CH_2$ (Kp. 86 bis 88°C/980 mbar) und 105 g (0,275 mol) Ausgangsverbindung liefert. Die Ausbeute beträgt 70,7%, bezogen auf umgesetztes $ICF_2CF_2CH_2CH_2I$. Kernresonanzdaten:

$^{19}F$-NMR: 18,2 ppm ($CF_2I$), −30,0 ppm ($CF_2$).
$^1H$-NMR: 5,78—6,09 ppm ($CH=CH_2$, Multiplett).

Herstellung von 3-Iod-tetrafluorpropionsäure

In einen 2,5 Liter-Glaskolben mit Tropftrichter, Rührer und Thermometer gibt man 383,9 g (2,43 mol) Kaliumpermanganat und 1 Liter entsalztes Wasser, fügt unter Rühren 5,0 g (0,016 mol) $[(C_8H_{17})_2N(CH_3)_2]^+Cl^-$ als Phasentransfer-Katalysator hinzu und rührt 10 Minuten. Nun tropft man unter externer Kühlung 187 g (0,7365 mol) $ICF_2CF_2—CH=CH_2$ unter kräftigem Rühren innerhalb von 3 Stunden zu, wobei die Innentemperatur zwischen 15 und 20°C gehalten wird. Man rührt anschließend noch 4 Stunden bei Raumtemperatur, erhitzt die Mischung auf 70°C und filtriert mittels eines heizbaren Druckfilters den gebildeten Braunstein ab. Der Braunstein wird mit 1 Liter siedendem Wasser gerührt und nochmals filtriert. Die vereinigten Filtrate werden unter vermindertem Druck am Rotationsverdampfer zur Trockne eingeengt. Der Rückstand wird in eine Destillationsapparatur überführt und vorsichtig mit 500 ml konzentrierter Schwefelsäure versetzt (anfangs Schäumen durch $CO_2$-Entwicklung). Durch Vakuumdestillation werden 290,6 g (72,5% Ausbeute) reine 3-Iod-tetrafluorpropionsäure erhalten (Kp. 78°C/13 mbar): eine farblose Flüssigkeit, die sich am Licht langsam rosa färbt und bei Raumtemperatur kristallisiert.

C: 13,1%, H: 0,5%, F: 27,7%, I: 46,8%
(berechnet: C: 13,25%, H: 0,37%, F: 27,95%, I: 46,66%).
Kernresonanzdaten:
$^{19}F$-NMR: 18,0 ppm ($CF_2I$), −32,9 ppm ($CF_2$)
$^1H$-NMR: 12,5 ppm ($CO_2H$).

**Patentansprüche**

1. Verfahren zur Herstellung von 3-Iod-tetrafluorpropionsäure, dadurch gekennzeichnet, daß man in einer Eintopfreaktion ein Gemisch von Iod, Tetrafluorethen und Ethen in einem Molverhältnis von 1:(1,2—2):(1—1,2) bei einer Temperatur von 170 bis 200°C umsetzt und aus dem erhaltenen Reaktionsprodukt die Verbindung 1.4-Diiod-1.1.2.2-tetrafluorbutan gewinnt, diese Butan-Verbindung zur Abspaltung von Iodwasserstoff mit einer Base umsetzt und aus diesem Reaktionsprodukt die Verbindung 1-Iod-1.1.2.2-tetrafluor-3-buten gewinnt, diese Buten-Verbindung zur Überführung in die entsprechende Propionsäure mit einem Oxidationsmittel umsetzt und aus diesem Reaktionsprodukt die 3-Iod-2.2.3.3-tetrafluorpropionsäure gewinnt.

2. Verfahren nach Anspruch 1, dadurch gekennzeichnet, daß die Umsetzung der Butan-Verbindung mit einer Base in der Weise durchgeführt wird, daß man die Butan-Verbindung mit einer mindestens stöchiometrischen Menge an Kalium- oder Natriumhydroxid in Form einer etwa 10 bis 60 gew.-%igen Wäßrigen Lösung bei einer Temperatur von 20 bis 100°C und in Gegenwart von Phasentransfer-Katalysatoren in einer Menge von 0,1 bis 5 Mol-%, bezogen auf die Menge an Butan-Verbindung, umsetzt.

3. Verfahren nach Anspruch 1, dadurch gekennzeichnet daß die Umsetzung der Buten-Verbindung mit einem Oxidationsmittel in der Weise durchgeführt wird, daß man die Buten-Verbindung mit einer mindestens stöchiometrischen Menge an Kaliumpermanganat in Form einer etwa 10 bis 40 gew.-%igen wäßrigen Lösung oder Suspension bei einer Temperatur von 5 bis 50°C und in Gegenwart von Phasentransfer-Katalysatoren in einer Menge von 0,5 bis 5 Mol-%, bezogen auf die Menge an Buten-Verbindung, umsetzt.

**Revendications**

1. Procédé pour préparer l'acide iodo-3 tétrafluoropropionique, procédé caractérisé en ce que, par une méthode en un seul récipient, on fait réagir un mélange d'iode, de tétrafluoréthylène et d'éthylène dans un rapport molaire de 1:(1,2—2):(1—1,2) à une température de 170 à 200°C, on isole, du mélange réactionnel obtenu, le diiodo-1,4 tétrafluoro-1,1,2,2 butane, on fait réagir ce dérivé du butane, pour provoquer un enlèvement d'iodure d'hydrogène, avec une base, on isole, du mélange réactionnel obtenu, l'iodo-1 tétrafluoro-1,1,2,2 butène-3, on fait réagir ce dérivé du butène avec un agent d'oxydation pour le convertir en l'acide propionique correspondant et, à partir du mélange réactionnel obtenu, on isole l'acide iodo-3 tétrafluoro-2,2,3,3-propionique.

2. Procédé selon la revendication 1 caractérisé en ce qu'on effectue la réaction du dérivé du butane avec une base de la façon suivante: on fait réagir le dérivé du butane avec un quantité au moins stoechiométrique d'hydroxyde de potassium ou de sodium sous la forme d'une solution aqueuse d'une concentration comprise entre environ 10 et 60% en poids, à une température de 20 à 100°C et en présence de catalyseurs de transfert de phase en une quantité de 0,1 à 5% en mole par rapport à la quantité de dérivé du butane.

3. Procédé selon la revendication 1 caractérisé en ce qu'on effectue la réaction du butène avec un agent d'oxydation de la façon suivante: on fait réagir le dérivé du butène avec une quantité au moins stoechiométrique de permanganate de potassium sous la forme d'une solution ou d'une suspension aqueuse d'une concentration comprise entre environ 10 et 40% en poids, à une température de 5 à 50°C et en présence de catalyseurs de transfert de phase en une quantité de 0,5 à 5% en molespar rapport à la quantité du dérivé du butène.

**Claims**

1. A process for the preparation of 3-iodotetrafluoropropionic acid characterised by reacting iodine, tetrafluoroethylene and ethylene in a molar ratio of 1:(1.2 to 2):(1 to 1.2) at a temperature of 170 to 200°C in a one-pot reaction, and isolating from the resulting reaction product the compound 1,4-diiodo-1,1,2,2-tetrafluorobutane, reacting this butane compound with a base in order to eliminate hydrogen iodide, and isolating the compound 1-iodo-1,1,2,2,-tetrafluoro-3-butene from this reaction product, reacting this butene compound with an oxidising agent in order to convert it into the corresponding propionic acid, and isolating 3-iodo-2,2,3,3-tetrafluoropropionic acid from this reaction product.

2. The process as claimed in claim 1, characterised in that the reaction of the butane compound with a base is carried out by reacting the butane compound with at least a stoichiometric amount of potassium hydroxide or sodium hydroxide in the form of an approximately 10 to 60% strength by weight aqueous solution at a temperature of 20 to 100°C and in the presence of phase transfer catalysts in an amount of 0.1 to 5 mol %, relative to the amount of butane compound.

3. The process as claimed in claim 1, characterised in that the reaction of the butene compound with an oxidising agent is carried out by reacting the butene compound with at least a stoichiometric amount of potassium permanganate in the form of an approximately 10 to 40% strength by weight aqueous solution or suspension at a temperature of 5 to 50°C and in the presence of phase transfer catalysts in an amount of 0.5 to 5 mol%, relative to the amount of butene compound.